Europäisches Patentamt

European Patent Office

Office européen des brevets

(11) Publication number: **0 360 205**
**A2**

## (12) EUROPEAN PATENT APPLICATION

(21) Application number: 89117271.0

(51) Int. Cl.5: **C12N 5/00**

(22) Date of filing: 19.09.89

(30) Priority: 19.09.88 JP 232529/88

(43) Date of publication of application:
28.03.90 Bulletin 90/13

(84) Designated Contracting States:
**DE FR GB IT NL**

(71) Applicant: **Fujimoto, Shigeyoshi**
**No. 587-75, A-206, Kamohara Okuo-cho**
**Nankoku-shi Kohchi-ken(JP)**

Applicant: **AJINOMOTO CO., INC.**
**5-8, Kyobashi 1-chome, Chuo-ku**
**Tokyo 104(JP)**

(72) Inventor: **Fujimoto, Shigeyoshi**
**No. 587-75, A-206, Kamohara Okou-cho**
**Nankoku-shi Kohchi-ken(JP)**

(74) Representative: **Türk, Gille, Hrabal**
**Bruckner Strasse 20**
**D-4000 Düsseldorf 13(DE)**

(54) Method for the induction and activation of cytotoxic T cells.

(57) The use of a serum-free medium for the induction of human self cancer-specific cytotoxic T cells (CTL) is disclosed.

EP 0 360 205 A2

## METHOD FOR THE INDUCTION AND ACTIVATION OF CYTOTOXIC T CELLS

## BACKGROUND OF THE INVENTION

Field of the Invention:

The present invention relates to the induction and activation of cytotoxic T cells.

Discussion of the Prior Art

It is known that cytotoxic T cells specifically recognize cancer cells or virus-infected cells by their antigen receptors, and impair these cells. Human self cancer-specific cytotoxic T cells (CTL) are important in view of their involvement in the in vivo mechanism of protecting the living body against cancer or viruses.

In recent years, it has been found that a group of cells which display cytotoxicity against cancer cells can be induced by culturing lymphocytes (collected from peripheral blood) in vitro together with interleukin 2 (IL-2). The so-called LAK therapy relates to the treatment of cancer by returning these cells to a living body.

LAK cells induced by this therapy however has no specificity towards individual cancer cells. Furthermore when these cells are administered to a living body, efficient accumulation of the cells onto the cancer-bearing tissue can not be achieved. Therefore, with LAK therapy a therapeutic effect is only exhibited by large doses administration of LAK cells, and, together with this, it is necessary to simultaneously administer IL-2. Since serious undesirable side effects are often observed under these circumstances, the administration of LAK cells is not a satisfactory therapy.

The present inventor has already established CTL therapy; a new immunotherapy exhibiting remarkable therapeutic effects [1, 2]. CTL therapy refers to the treatment of cancer by inactivating suppressor cells in vivo with (an) anticancer agent(s).

In this therapy lymphocytes are collected from the peripheral blood of the patient. The lymphocytes are cultured for 3 to 4 days together with cancer cells from the self cancer cells which have been treated with mitomycin C, etc. to suppress their ability to divide and proliferate. IL-2 is then added, and the mixture is cultured for an additional 3 to 4 days to proliferate and activate the CTL. The CTL are then administered to a living body.

The CTL activated by this method specifically recognize individual cancer cells and exhibit cytotoxicity. When these CTL are administered to a living body, they efficiently accumulate onto cancer-bearing tissue. In terms of their therapeutic effect, these CTL cells are administered in a smaller dose than are LAK cells, and it is unnecessary to simultaneously administer IL-2 in a large dose with them as is often required in LAK therapy.

Accordingly, the serious undesirable side effects of LAK therapy are not observed in CTL therapy. Furthermore, it was found that CTL therapy was also effective for involution to the metastatic focus of patients at the terminal stage of cancer. [3]

For inducing human self cancer-specific cytotoxic T cells from a human patient with cancer and activating the cells, with the prior art it was necessary to supplement Dulbecco's MEM (DMEM) with 5% human AB serum or 5% serum from umbilical cord blood. ("AB" indicates one type of "ABO" blood group system.) This approach suffers from the fact that there are great differences between different lots of sera. One serum batch could be capable of inducing CTL relatively efficiently, while another serum batch could conversely inhibit CTL induction or make CTL induction impossible, etc.

It is unclear what causes these differences in quality. But even if a serum capable of inducing CTL efficiently could be obtained, the amount of serum which could be supplied from the same lot is extremely limited. It is thus difficult to induce and activate CTL efficiently with good reproducibility. Furthermore, as long as serum of human origin is used, it is impossible to eliminate the danger of pathogenic contaminants such as viruses, etc. Accordingly, in applying CTL induced and activated by this method to CTL therapy, many problems are encountered.

There is thus a distinct need for a method for inducing stable CTL having a high activity in CTL therapy, and in which there are no dangers of pathogenic contaminants such as viruses, etc.

## SUMMARY OF THE INVENTION

Accordingly it is an object of this invention to provide a method for the induction and activation of cytotoxic T cells which does not suffer from the disadvantages noted above. The inventor has now discovered a method which satisfies this object and other objects as can be seen from the description given below. This method comprises using a serum-free medium to cause the induction of human self cancer-specific cytotoxic T cells.

The invention also provides a kit for the induction and activation of cytotoxic T cells. This kit comprises a serum-free medium and a means for culturing cells in a suspension culture.

## DETAILED DESCRIPTION OF THE PREFERRED EMBODIMENTS

CTL are useful for the treatment of cancer or viral diseases such as HTLV-III, etc. In CTL therapy, CTL are administered to a living body.

The inventor has made extensive investigations. As a result, it has been found that a serum-free medium obtained by incorporating various growth factors (such as insulin, transferrin, steroid hormones, etc. or ethanolamine, human serum albumin, etc.) into a modified medium such as, e.g., RPMI 1640® medium, Ham F-12® medium, DMEM®medium, [3] etc. which does not substantially contain serum, singly or admixtures thereof, etc., as a basic medium, is very well suited for the induction and activation of human cancer cell-specific cytotoxic T cells. Insulin may be used in an amount of from 0.1 micrograms $ml^{-1}$ to 100 micrograms $ml^{-1}$. Transferin may be used in an amount of from 0.1 micrograms $ml^{-1}$ to 1,000 micrograms $ml^{-1}$. Steroid hormones may be used in an amount of from 1 nanogram $ml^{-1}$

([3] Available from GIBCO Oriental Co. Inc., Nihonhashi Kodenma-cho 10-11, Chuoh-ku, Tokyo, Japan, 103.) to 1 microgram $ml^{-1}$. The present invention thus provides using such a serum-free medium which contains, as its basic elements and in addition to the components described above, sugar, amino acids, vitamins, heavy metals, pH buffers, etc. to induce and activate cytotoxic T cells.

In a preferred embodiment one preferably uses crystallized insulin of human, bovine or swine origin as the insulin component, and one uses transferrin and albumin of human origin. It is even more desirable to use recombinant human insulin, recombinant human transferring and/or recombinant human albumin.

The serum-free medium used in the present invention is not limited to the examples set out above. Other specific examples of the serum-free medium in the present invention include ASF-104® manufactured by Ajinomoto Co., Inc., of 5-8, = Kyobashi, 1-chome Chuo-ku, Tokyo, Japan 104, HB104®, KC2000®, SFM-101®, COSMEDIUM-001®, etc.

The serum-free media which are used in the present invention are much more potent in the generation of normally functioning CTL specific for self cancer cells than are ordinary media such as DMEM and RPMI 1640. This is shown by the data tabulated in Tables 1 and 3, infra.

These serum-free media do not require the presence of various serum proteins with the exception of 1% albumin as a protein supplement for specific CTL generation. By contrast ordinary media, such as DMEM and RPMI 1640, require the presence of at least 5% whole serum proteins for this purpose.

For the induction and activation of human cancer cell-specific CTL using the serum-free medium of the present invention, a container or device routinely used for conventional suspension cell culture may be used.

In the present invention self cancer cells are pretreated with mitomycin C, etc. to prevent their ability to divide and proliferate. These are then added together with lymphocytes isolated from the peripheral blood of a patient to the serum-free medium described above. Cell densities of from 3 to 5 x $10^6$ $ml^{-1}$ and from 1 to 5 x $10^4$ $ml^{-1}$, respectively, are used.

This is followed by culturing at 37°C for 2 to 4 days under 5% $CO_2$. Thereafter, IL-2 (e.g., human recombinant IL-2) is added to the medium at a concentration of 25 to 75 ng $ml^{-1}$. This is followed by culturing for another 2 to 4 days to obtain efficient induction and activation of CTL specific to self cancer cells.

To this culture, homologous cancer cells in which at least a part of the human leucocyte antigen (HLA) type is identical may also be used as stimulator cells instead of the self cancer cells. Further at the initiation of culture, an immunoactivator or a biological response modifier, specifically, OK432 (streptococcal preparation), Nocardia (Nocardia cell wall preparation), Bestatis (Uvenimex), etc. may also be added in a suitable amount. For example, OK432 may be used in an amount of from 0.001 to 0.05 Ka $ml^{-1}$ (Klinische

Einhait) equivalent to 0.01 micrograms to 5 micrograms $ml^{-1}$. Norcardia CWS can be used in an amount of from 10 micrograms to 50 micrograms $ml^{-1}$ and Bestatin can be used in an amount of from 10 micrograms to 400 micrograms $ml^{-1}$.

To determine whether induction of CTL to self cancer cells has occurred, the conventional chromium release method may be used. That is, a 20-fold, 40-fold or 80-fold amount of CTL is added to $1 \times 10^3$ of self cancer cells marked with chromium 51. After culturing at $37^\circ$C for 16 hours under 7% $CO_2$, the amount of chromium 51 released in the supernatant is measured with a gamma counter to determine CTL.

When CTL specific to self cancer cells from lymphocytes collected from the peripheral blood of a patient with cancer are induced and activated according to the present invention, CTL having an activity as potent as about 10 times that obtained by inducing CTL by the conventional method of using serum medium is obtained with good reproducibility. Furthermore, because the present invention uses a serum-free medium, it is free of problems such as differences between lots depending upon serum, limited amounts of supply, danger of pathogenic contaminants such as viruses, etc.

A list of cancer types which have been treated by CTL therapy is provided below.

| SARCOMA | BRAIN TUMOR | CARCINOMA |
|---|---|---|
| Epithelioid Sarcoma<br>Synovial Sarcoma<br>Osteosarcoma<br>Malignant Schwannoma<br>Malignant Melanoma<br>Leiomyosarcoma | Glioblastoma<br>Astrocytoma<br>Medulloblastoma | Breast cancer<br>Ovarian cancer<br>Lung cancer<br>Gastric cancer<br>Colon cancer<br>Thyroid cancer<br>Pharyngeal cancer<br>Cervical cancer<br>Renal cancer |

CTL therapy can be used against any cancer in which the patient's CTL can be generated by in vitro stimulation with self-cancer cells. Generated CTL can be administered to a patient undergoing CTL therapy at a dosage ranging from $1 \times 10^7$ to $5 \times 10^8$ cells per administration. Generally one administration per week is used. A total of 10 to 30 administrations is generally used.

Having generally described this invention, a further understanding can be obtained by reference to certain specific examples which are provided herein for purposes of illustration only and are not intended to be limiting unless otherwise specified.

Example 1

Lymphocytes obtained from the peripheral blood of a patient with epitheloid cell sarcoma were added, singly, or together with self epitheloid cell sarcoma in which the ability of division and proliferation was prevented by treatment with 100 $\mu g$ $ml^{-1}$ of mitomycin C for an hour, to 30 ml of ASF-104 medium supplemented with 1% human serum albumin or to 30 ml of DMEM medium supplemented with 1% human serum albumin or 5% human serum as shown in Table 2, in cell densities of $3 \times 10^6$ $ml^{-1}$ and $3 \times 10^4$ $ml^{-1}$, respectively. After culturing in a T-shaped flask having a bottom area of 75 $cm^2$ manufactured by Nunc Inc. for 3 days, human recombinant IL-2 was added in a concentration of 57.1 ng $ml^{-1}$. By culturing for another 3 days, CTL specific to self epitheloid cell sarcoma was induced.

The cytotoxic activity to self cancer cells after the induction was assayed by the chromium 51 release method in a conventional manner. That is, $4 \times 10^4$ or $8 \times 10^4$ counts of CTL was added to $1 \times 10^3$ of self epitheloid sarcoma marked with chromium 51. After culturing at $37^\circ$C for 16 hours under 7% $CO_2$, the amount of chromium 51 released in the supernatant was measured with a gamma counter. The killer activity value was calculated by the following equation:

Killer activity (%) = $100 \times (X - [B])/([A] - [B])$

In the above equation:

[A] represents the amount of chromium 51 released in the medium when $1 \times 10^3$ of chromium-labeled epitheloid sarcoma cells were completely dissolved in 2.5% saponin solution;

[B] represents the amount of chromium 51 released in the medium when $1 \times 10^3$ of epitheloid sarcoma cells alone were cultured for 16 hours; and

4

X represents the amount of chromium 51 released in the medium when cultured together with CTL induced by the above method.

The results obtained are shown in Table 1.

Table 1.

| Killer activity on epitheloid sarcoma cells | | | |
|---|---|---|---|
| Medium | Cancer Cells Treated with MMC | Killer Activity | |
| | | 80 : 1 | 40 : 1 |
| ASF-104 | - | 32.6 ± 3.0 | 23.4 ± 1.4 |
| + 1% human albumin | + | 51.4 ± 3.8 | 32.0 ± 3.5 |
| DMEM | - | 18.6 ± 1.2 | 11.9 ± 1.8 |
| + 1% human albumin | + | 15.7 ± 1.9 | 8.4 ± 2.4 |
| DMEM | - | -1.0 ± 1.4 | -1.9 ± 2.6 |
| + 5% human serum | + | 4.7 ± 1.5 | 0.9 ± 2.3 |

Table 2

| Reagent | Content (g/set) |
|---|---|
| Sodium chloride | 60 |
| Potassium chloride | 4 |
| Calcium chloride (anhydrous) | 1 |
| Magnesium sulfate (anhydrous) | 0.98 |
| Sodium dihydrogenphosphate (anhydrous) | 1.25 |
| Ferric nitrate (9 hydrate) | 0.001 |
| Zinc sulfate (7 hydrate) | 0.0001 |
| Sodium selenite | 0.00004 |
| Copper sulfate (5 hydrate) | 0.00001 |

| | |
|---|---|
| Glucose | 20 |
| Mannose | 5 |
| Galactose | 2 |
| α-CD | 20 |
| L-arginine hydrochloride | 2 |
| L-alanine | 0.2 |
| L-asparagine (1 hydrate) | 0.56 |
| L-aspartic acid | 0.2 |
| Sodium L-glutamate | 0.2 |
| Glycine | 0.3 |
| L-histidine hydrochloride (1 hydrate) | 0.42 |
| L-isoleucine | 1.05 |
| L-leucine | 1.05 |
| L-lysine hydrochloride | 1.46 |
| L-methionine | 0.3 |
| L-phenylalanine | 0.67 |
| L-proline | 0.2 |
| L-serine | 0.8 |
| L-ornithine hydrochloride | 1 |
| L-threonine | 0.85 |
| L-tryptophan | 0.25 |
| L-tyrosine | 0.64 |
| L-valine | 0.94 |
| Glycyl-glutamine | 5.0 |
| Alanyl-glutamine | 5.0 |
| Antibiotic (kanamycin) | 0.6 |

| | |
|---|---|
| Succinic acid | 1.06 |
| Sodium succinate (6 hydrate) | 0.27 |
| Phenol red (for medium) | 0.05 |
| Deoxyadenosine | 0.01 |
| Deoxycytidine | 0.0003 |
| 6,8-Dihydroxypurine | 0.003 |
| Thymidine | 0.002 |
| Uridine | 0.05 |
| Biotin | 0.002 |
| Calcium pantothenate | 0.04 |
| Choline tartarate | 0.2 |
| Vitamin B12 | 0.001 |
| Folic acid | 0.04 |
| Calcium fornate | 0.0001 |
| i-Inositol | 0.2 |
| Nicotinic amide | 0.04 |
| Pyridoxal hydrochloride | 0.04 |
| Riboflavin | 0.004 |
| Thiamine hydrochloride | 0.04 |
| Disodium hypoxanthate | 0.02 |
| Disodium β-glycerophosphate | 15 |
| HEPES (free) | 12 |
| Sodium hydrogencarbonate | 18 |
| Human transferrin | 0.05 |
| Sodium pyruvate | 2.2 |
| Ferrous sulfate (1 hydrate) | 0.7 |

7

| | |
|---|---|
| Crystalline insulin | 0.05 |
| Glutathione (reductive) | 0.01 |
| Sodium ascorbate | 0.05 |
| Putrescine dihydrochloride | 0.003 |
| α-CD | 2 |
| α-CD-linoleic acid | 0.1 |

Example 2

Lymphocytes obtained from the peripheral blood of a patient with osteosarcoma were added, singly, or together with self osteosarcoma in which the ability of division and proliferation was prevented by treating with 100 μg/ml of mitomycin C for an hour, to 30 ml of ASF-104 medium supplemented with 1% human serum albumin, to 30 ml of DMEM medium supplemented with 1% human serum albumin, or to 30 ml of RPMI 1640 medium supplemented with 1% human serum albumin shown in Table 2, in cell densities of 3 x $10^6$ ml$^{-1}$ and 3 x $10^4$ ml$^{-1}$, respectively, in the presence of or absence of 0.005 KE ml$^{-1}$ of OK432. After culturing in a T-shaped flask having a bottom area of 75 cm$^2$ manufactured by Nunc Inc. for 3 days, human recombinant IL-2 was added in a concentration of 57.1 ng ml$^{-1}$. By culturing for another 3 days, CTL specific to self osteosarcoma was induced.

The cytotoxic activity to self osteosarcoma cells after the induction was assayed by the chromium 51 release method for 16 hours in a conventional manner. The results are shown in Table 3.

Table 3.

| Killer activity on osteosarcoma cells | | | | |
|---|---|---|---|---|
| Medium | OK432 (0.05 KE ml) | Cancer Cells Treated with MMC | Killer Activity | |
| | | | 80 : 1 | 40 : 1 |
| ASF-104 | - | - | 24.7 ± 1.7 | 18.1 ± 1.6 |
| + 1% human albumin | - | + | 38.2 ± 1.0 | 20.8 ± 1.9 |
| DMEM | + | + | 88.7 ± 2.1 | 80.1 ± 1.5 |
| + 1% human albumin | - | - | 2.9 ± 1.7 | 4.4 ± 3.0 |
| RPMI 1640 | - | + | 0.7 ± 1.1 | -0.6 ± 0.8 |
| + 5% human serum | - | - | 3.7 ± 0.6 | 1.8 ± 0.8 |
| | - | + | 2.9 ± 1.2 | 0.4 ± 1.3 |

DOCUMENTS CITED IN THE TEXT

1. Fujimoto et al, Report in the Proceeding of the Japanese Cancer Association, 46th Annual Meeting, September 1987 (Tokyo), page 311.

8

2. Fujimoto et al, Report in the Proceedings of the Japanese Society for Immunology, volume 17, page 539.

3. Fujimoto et al, Abstract of the 6th General Meeting on Human Cell Study in 1988, page 124.

Obviously, numerous modifications and variations of the present invention are possible in light of the above teachings. It is therefore to be understood that within the scope of the appended claims, the invention may be practiced otherwise than as specifically described herein.

## Claims

1. In a method for the induction and activation of cytotoxic T cells, the improvement comprising using a serum-free medium to cause the induction of human self cancer-specific cytotoxic T cells.

2. The method of Claim 1, comprising adding an immunoactivator to said serum-free medium.

3. The method of Claim 1, comprising adding a growth factor to said serum-free medium.

4. The method of Claim 3, wherein said growth factor is at least one member selected from the group consisting of insulin, transferin, steroid hormones, ethanol amine, and human serum albumin.

5. The method of Claim 4, comprising using insulin of human, bovine or swine origin as the insulin component.

6. The method of Claim 4, comprising using transferin of human origin.

7. The method of Claim 4, comprising using recombinant human insulin, recombinant human transferin or recombinant human albumin.

8. A method for the induction and activation of cytotoxic T cells, comprising:

(i) combining (ia) self cancer cells unable to divide and proliferate with (ib) lymphocytes isolated from the peripheral blood of a patient, and (ic) a serum-free medium, to obtain a composition having a cell density of from 3 to 5 x $10^6$ ml$^{-1}$ and from 1 to 5 x $10^4$ ml$^{-1}$, respectively;

(ii) culturing said composition for 2 to 4 days under an atmosphere of carbon dioxide;

(iii) adding interleukin-2 to the medium at a concentration of 25 to 75 ng ml$^{-1}$;

(iv) culturing said medium for an additional 2 to 4 days; and

(v) obtaining induced inactivated CTL specific to said self cancer cells.

9. The method of Claim 8, comprising using, instead of said self cancer cells, homologous cancer cells in which at least a part of the human leukocyte antigen type is identical.

10. A kit for the induction and activation of cytotoxic T cells comprising a serum-free medium and a means for culturing cells in a suspension culture.

11. The kit of Claim 10, wherein said serum-free medium comprises a growth factor.

12. The kit of Claim 11, wherein said growth factor is at least one member selected from the group consisting of insulin, transferin, steroid hormones, ethanol amine, and human serum albumin.

13. The kit of Claim 12, comprising insulin of human, bovine or swine origin as said insulin component.

14. The kit of Claim 12, comprising transferin of human origin.

15. The kit of Claim 12, comprising recombinant human insulin.

16. The kit of Claim 12, comprising recombinant human transferin.

17. The kit of Claim 12, comprising recombinant human albumin.